# EUROPEAN PATENT SPECIFICATION

(11) **EP 1 732 574 B1**
(45) Date of publication and mention of the grant of the patent: **27.01.2010**
(21) Application number: 05707370.2
(22) Date of filing: 14.02.2005
(51) Int. Cl.: A61K 31/7076, A61K 45/06, A61P 27/02, A61P 27/06, A61P 9/10, A61P 35/00, A61P 35/02

(54) **USE OF O-ATP FOR THE TREATMENT OF DISEASES INVOLVING ANGIOGENESIS**
VERWENDUNG VON O-ATP FÜR DIE BEHANDLUNG VON ANGIOGENESE-BEZOGENEN KRANKHEITEN
UTILISATION DE O-ATP POUR LE TRAITEMENT DE MALADIES IMPLIQUANT UNE ANGIOGENESE

(30) Priority: 17.02.2004 IT MI20040025
(43) Date of publication of application: 20.12.2006
(73) Proprietor: Medestea Internazionale S.p.A., 10121 Torino (IT)
(72) Inventor: FERRERO, Maria Elena, I-20149 Milano (IT)
(74) Representative: Banfi, Paolo
(86) International application number: PCT/EP2005/001458
(87) International publication number: WO 2005/077383

(56) References cited:
- EP-A- 1 310 493
- WO-A-02/11737
- WO-A-92/08456
- WO-A-03/042191
- US-A1- 2005 053 612
- CORY J G ET AL: "IRREVERSIBLE INHIBITION OF RIBO NUCLEOTIDE REDUCTASE FROM EHRLICH TUMOR CELLS BY A MODULATOR ANALOGUE" BIOCHEMICAL AND BIOPHYSICAL RESEARCH COMMUNICATIONS, vol. 52, no. 2, 1973, pages 496-503, XP008048752 ISSN: 0006-291X
- CORY J G ET AL: "INHIBITION OF RNA SYNTHESIS IN EHRLICH TUMOR CELLS BY THE DI ALDEHYDE DERIVATIVE OF INOSINE INOX NSC-118994" CANCER RESEARCH, vol. 38, no. 3, 1978, pages 815-822, XP008048769 ISSN: 0008-5472
- SUGIYAMA T ET AL: "Activation of P2X7 Receptors as a Death Signal in the Normal and Diabetic Retinal Microvasculature." ARVO ANNUAL MEETING ABSTRACT SEARCH AND PROGRAM PLANNER, vol. 2002, 2002, page Abstract No. 1340, XP008048761 & ANNUAL MEETING OF THE ASSOCIATION FOR RESEARCH IN VISION AND OPHTHALMOLOGY; FORT LAUDERDALE, FLORIDA, USA; MAY 05-10, 2002
- BEIGI REZA D ET AL: "Oxidized ATP ( oATP ) attenuates proinflammatory signaling via P2 receptor-independent mechanisms." BRITISH JOURNAL OF PHARMACOLOGY, (2003 OCT) 140 (3) 507-19. JOURNAL CODE: 7502536. ISSN: 0007-1188., October 2003 (2003-10), XP008048760
- RICCHI P ET AL: "Nonsteroidal anti-inflammatory drugs in colorectal cancer: From prevention to therapy." BRITISH JOURNAL OF CANCER, vol. 88, no. 6, 24 March 2003 (2003-03-24), pages 803-807, XP008048756 ISSN: 0007-0920
- ZHANG XIULAN ET AL: "Stimulation of P2X7 Receptors Elevates Ca2+ and Kills Retinal Ganglion Cells." INVESTIGATIVE OPHTHALMOLOGY & VISUAL SCIENCE. JUN 2005, vol. 46, no. 6, June 2005 (2005-06), pages 2183-2191, XP008048763 ISSN: 0146-0404
- DATABASE BIOSIS BIOSCIENCES INFORMATION SERVICE, PHILADELPHIA, PA, US; & Pharm. Biol., 41(8), 604-607 2003, RAJKAPOOR B. ET AL.: "Antitumor activity of Buhinia variegata against Ehrlich ascites carcinoma indiced mice"

## Description

The present invention relates in general to substances that act on angiogenesis. More precisely, the invention relates to the use of o-ATP for the treatment of pathologies that require inhibition of angiogenesis.

### BACKGROUND OF THE INVENTION

### Angiogenesis

Proliferation of endothelial cells is resposible for the process of formation of new blood vessels, known as angiogenesis. The newly formed vessels provide nutrients and oxygen to the cells of the tissue wherein angiogenesis occurs. The angiogenetic process is useful, for example, for wound repair, since regenerating tissues necessitate a proper blood supply. On the contrary, angiogenesis is detrimental in the case of tumour diseases, because blood supply facilitates the proliferation of tumour cells. In addition, neoangiogenesis is detrimental when develops into the atherosclerotic plaques; in fact in these structures the generation of new vessels due to VEGF (vascular endothelial growth factor) production by endothelial and other cells, as monocytes/macrophages, supports the preservation of the same plaques. Therefore, the inhibition of endothelial cells proliferation, or anti-angiogenic activity, is of remarkable interest in antitumour and antiatherosclerotic therapies.

### o-ATP's Biological activity

The oxidized form of ATP, known as o-ATP, is characterised by the presence of two aldheyde groups at the positions 2' and 3' of the ribofuranosyl ring. It can be prepared by treatment of ATP with a periodic acid salt, as disclosed by P.N. Lowe et al., "Preparation and chemical properties of periodate-oxidized adenosine triphosphate and some related compounds", Biochemical Society Transactions, vol. 7:1131-1133, 1979.

o-ATP is commonly used as an affinity marker for nucleotide enzymatic sites (Easterbrook-Smith, B., Wallace, J.C. & Keech, D.B. (1976) Eur. J. Biochem. 62, 125-130), thanks to its ability to react with non-protonated lysine residues that are present in the nucleotide sites, forming Schiff's bases or dihydromorpholine-derivatives (Colman, R.F. (1990) in The Enzymes - Sigman, D.S., and Boyer, P.D., eds - Vol 19, pp. 283-323, Academic Press, San Diego). It has also been used to study platelet activation and inhibit ATP-induced stimulation of chicken muscle (Pearce, P.H., Wright, J.M. Egan. C.M. & Scrutton, M.C. (1978) Eur. J. Biochem. 88, 543-554; Thomas, S.A., Zawisa, M.J., Lin, X. & Hume, R.I. (1991) Br. J. Pharmacol. 103, 1963-1969). Furthermore, in macrophage cell lines, o-ATP proved able to block ATP-induced permeabilization of the plasma membrane, reduce the hydrolysis level of exogenous ATP by membrane ecto-ATPases, and inhibit ATP-induced cell swelling, vacuolization and lysis (Murgia et al. The Journal of Biological Chemistry, (1993) by The American Society for Biochemistry and Molecular Biology, inc., Vol. 268, No. 11, pp 8199). It has been suggested that o-ATP has an antagonist activity on the purinergic receptor P2z/P2X7, due to the fact that IL-1β (interleukin 1β) release (which is dependent on LPS = lipopolysaccharide) from microglia cells expressing P22/P2X7 is selectively inhibited by o-ATP (Ferrari D. et al., J. Exp. Med., (1997) Vol. 185, N. 3, Pag. 579-582).

### PRIOR ART

WO 02/11737, in the name of the Applicant, discloses o-ATP antinflammatory and analgesic effect, using unilateral inflammation of rat paw caused by intraplantar injection of complete Freund's adjuvant (CFA) as the experimental model.

WO-A-9208456 discloses the use of compounds which inhibit creatine kinase or other enzymes which participate in purine metabolism for the treatment or prevention of tumours characterised by elevated levels of these enzymes. The compound can be a 2',3'-dialdehyde derivative of ATP, which corresponds to o-ATP.

Gory et al. report in Biochem, Biophys. Res. Comm., 52(2), 496-503, 1973 that the dialdehyde derivative of ATP (= o-ATP) is an irreversible inhibitor of ribonucleotide reductase from Ehrlich tumor cells, and in Cancer Res., 38(3), 815-822, 1978 that the dialdehyde derivative of ATP inhibits RNA synthesis in isolated nuclei of Ehrlich tumor cells.

### DESCRIPTION OF THE INVENTION

In vitro assays on human umbilical vein endothelial cells (HUVEC), have shown that o-ATP induces a significant reduction of their proliferative capacity, even in the presence of a mitogen. The effect of o-ATP resulted higher than that induced by vasostatin, a known anti-angiogenic compound.

The use of o-ATP for the inhibition of angiogenesis as specified in the claims is therefore object of the present invention. In particular, the invention provides a medicament containing o-ATP as the active principle, useful for the treatment of pathologies as specified in the claims, the onset or progression of which involves angiogenesis. The angiogenesis-mediated diseases that can benefit from the treatment with o-ATP according to the invention are atherosclerotic processes and tumours selected from lymphomas and leukaemia.

For therapeutical use, o-ATP can be formulated with pharmaceutically acceptable carriers and excipients, and administered through the oral, topical or parenteral route. Pharmaceutical forms suitable for the different administration routes comprise tablets, pills, capsules, granulates, powders, suppositories, syrups, solutions, suspensions, creams, ointments, gels, pastes, lotions, emulsions, sprays. Pharmaceutical compositions can be prepared as described in Remington's Pharmaceutical Sciences Handbook, Mack Pub. Co., NY, USA, XVII Ed. The amount of active substance per dose unit ranges from 0.01 to 100 mg per Kg of body weight, to be administered once a day or more according to the type and severity of the pathology. In general the daily dose will range from 1 to 300 mg, preferably from 10 to 100 mg.

In another embodiment, the invention refers to combined preparations of o-ATP and other biologically active substances for the treatment of angiogenesis-mediated pathologies as specified in the claims. According to a preferred embodiment, o-ATP is used in combination with antiatherosclerotic substances selected from lipid lowering drugs or statins.

The different active substances can be administered either simultaneously or separately. The choice of the specific combination of active substances, their dosage and way of administration depend on the specific disease, its resistance to pharmacological treatments, patient's tolerance and other variables to be determined on a case by case basis.

### Example 1 - Proliferation assay

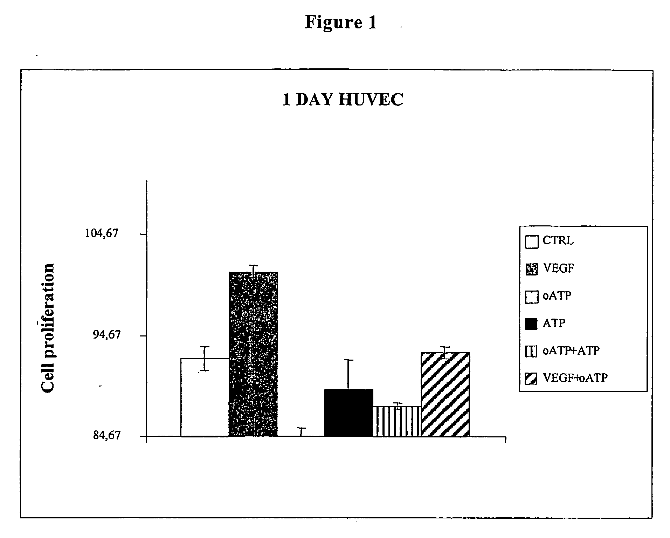

Human endothelial Cells (HUVEC) were isolated from umbilical vein, counted and seeded in a costant number in a 96-wells plates. The cells were cultured as described (Jaffe, E.A. (1984) Biology of Endothelial Cells, Martinus Nighoff Publisher, Boston, USA, pp. 1-260), with or without (control) VEGF (50 ng/ml), in the presence of o-ATP (100 µM), and o-ATP+VEGF. After 24 hours cultivation with or without stimulus, the cells were washed and counted with an optical microscope using a Burker chamber. The results are reported in Figure 1 and represent the mean ± SD of 10 experiments.

### Example 2 - Permeability assay

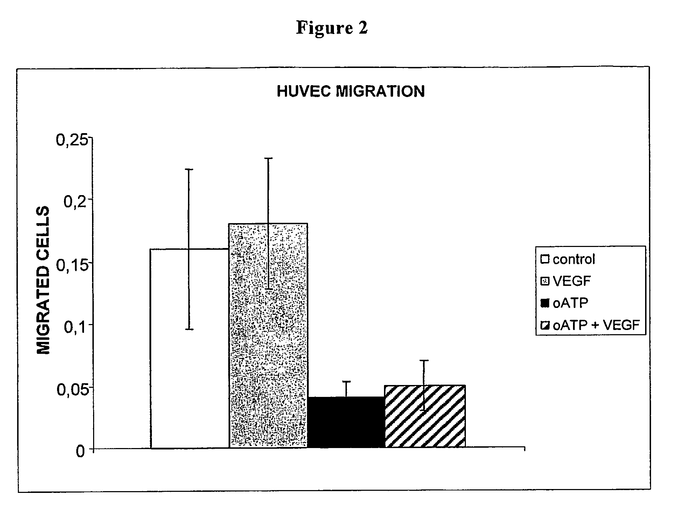

Transwell chambers for cell cultures (polycarbonate filters 0.4 µm, Costar) were used. In short, confluent endothelial cells, in monolayer, were exposed to VEGF, o-ATP, ATP (300 µM), ATP+o-ATP, o-ATP+VEGF (at the previously indicated concentrations) for 1 hr and thoroughly washed. Albumin marked with ¹²⁵I (NEN, Boston, MA) was added to the upper compartment; cold albumin (1.5 mg/ml) was added to the culture medium to minimize transcytosis. One hour after the addition of ¹²⁵I-labelled albumin to each well, samples were taken from the lower compartment. The radioactivity of the samples was measured with a gamma counter (Packard, Sterling, VA). The results, reported in Figure 2, represent the mean ± SD of 10 independent experiments and are expressed as percentage of migrated endothelial cells.

## Claims

1. The compound o-ATP for use in the treatment of a disease selected from atherosclerosis, lymphoma and leukaemia.

2. Therapeutic preparation containing o-ATP in combination with an antiatherosclerotic substance selected from lipid lowering drugs and statins, for the simultaneous, separate or sequential use in the treatment of atherosclerotic processes.

## Patentansprüche

1. Die Verbindung o-ATP zur Verwendung bei der Behandlung einer Krankheit ausgewählt aus Atherosklerose, Lymphom und Leukämie.

2. Therapeutische Zubereitung enthaltend o-ATP in Kombination mit einer antiatheroskletotischen Substanz ausgewählt aus Lipid-senkenden Arzneimitteln und Statinen, für die gleichzeitige, getrennte oder aufeinanderfolgende Verwendung bei der Behandlung von atheroskletotischen Prozessen.

## Revendications

1. Composé o-ATP pour son utilisation dans le traitement d'une maladie choisie parmi l'athérosclérose, le lymphome et la leucémie.

2. Préparation thérapeutique contenant de l'o-ATP en combinaison avec une substance antiathérosclérotique choisie parmi médicaments hypolipidémiants et les statines pour l'utilisation simultanée, séparée ou séquentielle dans le traitement des processus athérosclérotiques.
